# EUROPÄISCHE PATENTSCHRIFT

(11) **EP 0 843 659 B1**
(45) Veröffentlichungstag und Bekanntmachung des Hinweises auf die Patenterteilung: **14.04.1999**
(21) Anmeldenummer: 96927681.5
(22) Anmeldetag: 05.08.1996
(51) Int. Cl.: C07C 291/04, C11D 1/75

(54) **POLYHYDROXYALKYL-AMIDAMINOXIDE**
POLYHYDROXY ALKYL AMIDAMINE OXIDES
OXYDES DE POLYHYDROXYALKYLAMIDAMINES

(30) Priorität: 11.08.1995 DE 19529466
(43) Veröffentlichungstag der Anmeldung: 27.05.1998
(73) Patentinhaber: Clariant GmbH, 65929 Frankfurt am Main (DE)
(72) Erfinder: PAPENFUHS, Bernd, D-84524 Neuötting (DE)
(86) Internationale Anmeldenummer: EP9603445
(87) Internationale Veröffentlichungsnummer: WO9707094

(56) Entgegenhaltungen:
- US-A- 4 077 990
- US-A- 4 864 060

## Beschreibung

Die Erfindung betrifft Polyhydroxyalkyl-amidaminoxide, wäßrige, alkoholische oder wäßrig-alkoholische Lösungen davon, ein Verfahren zur Herstellung dieser Aminoxide und ihrer Lösungen sowie die Verwendung der neuen Aminoxidverbindungen und deren Lösungen.

Aminoxide sind wertvolle Verbindungen aus der Gruppe der zwitterionischen Tenside. Aufgrund ihres guten Reinigungsvermögens und ihrer weiteren vorteilhaften Eigenschaften, insbesondere hinsichtlich Schaumverhalten und Hautverträglichkeit, werden sie in Form von flüssigen Formulierungen vor allem zur Haar- und Körperreinigung eingesetzt. Die Lösungsmittel sind im allgemeinen Wasser, niedere Alkanole wie Methanol, Ethanol, Isopropanol, Ethylenglykol und/oder Propylenglykol oder eine Mischung davon.

Im Hinblick auf Lager- und Transportkosten, Weiterverarbeitung und Anwendung an Ort und Stelle sind konzentrierte bis hochkonzentrierte (möglichst wenig Lösungsmittel enthaltende) und gleichzeitig niedrigviskose Formulierungen erwünscht. Die im Handel befindlichen Aminoxidlösungen haben im allgemeinen einen Aminoxidgehalt (Wirkstoffgehalt) von weniger als 30 Gew.-%. Im Falle von 30 bis etwa 35 Gew.-% Wirkstoffgehalt spricht man von konzentrierten und bei noch höherem Wirkstoffgehalt von hochkonzentrierten Aminoxidlösungen.

Die Herstellung von Aminoxiden erfolgt im allgemeinen durch Oxidation von tertiären Aminverbindungen mit Wasserstoffperoxid in wäßrigem oder wäßrig-alkoholischem Medium.

Besonders im Falle von Amidaminoxiden, deren Anwendungsgebiete vor allem auf dem Kosmetik- und Reinigungsmittelsektor liegen (vergleiche US-A-4 077 990, JP-A-61-283695 und EP-A-367 926), ist die Löslichkeit stark eingeschränkt.

Es wurde nun eine neue Klasse von Amidaminoxiden gefunden, die sich durch eine gute Wasserlöslichkeit auszeichnen und auch niedrigviskose Formulierungen mit einer hohen Aminoxidkonzentration ergeben.

Die erfindungsgemäßen Aminoxide aus der Gruppe der oxidierten Polyhydroxyalkylamidamine entsprechen der nachstehenden Formel (1) 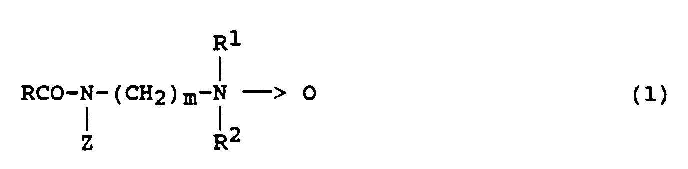 worin bedeuten
- RCO: einen aliphatischen Acylrest mit 6 bis 22 Kohlenstoffatomen,
- z: einen linearen Polyhydroxykohlenwasserstoffrest mit mindestens 3 gegebenenfalls oxalkylierten Hydroxylgruppen,
- m: eine ganze Zahl von 1 bis 4,
- R¹: C₁ bis C₄-Alkyl oder C₂ bis C₄-Hydroxyalkyl und
- R²: C₁ bis C₄-Alkyl oder C₂ bis C₄-Hydroxyalkyl.

Bevorzugte erfindungsgemäße Verbindungen der Formel (1) sind solche wobei
- RCO: ein Fettacylrest mit 8 bis 18 Kohlenstoffatomen ist,
- Z: ein Rest eines Zuckeralkohols ist, der sich von einem reduzierenden Mono- oder Disaccharid, insbesondere von Glucose, ableitet,
- m: für die Zahl 3 steht und
- R¹ und R²: (gleich oder verschieden) Methyl, Ethyl, Propyl oder Hydroxyethyl sind.

Zu RCO und Z sei noch folgendes gesagt: Der aliphatische Acylrest RCO, der vorzugsweise der genannte Fettacylrest ist, kann gesättigt oder ungesättigt (vorzugsweise ein- bis dreifach ungesättigt) sein. Als Beispiele seien die Acylreste von Capryl-, Caprin-, Laurin-, Palmitin-, Stearin- und ölsäure genannt sowie Cocosacyl, Talgacyl, vorzugsweise gehärtetes Talgacyl, und dergleichen. Der Fettsäurerest stellt häufig eine Mischung von zwei oder mehreren Acylgruppen dar, zum Beispiel C₁₂ und C₁₄-Acyl (C_{12/14}), C₁₆ und C₁₈-Acyl (C_{16/18}) oder C₁₂ bis C₁₈-Acyl. Der lineare Polyhydroxykohlenwasserstoffrest stammt, wie oben bereits erwähnt, vorzugsweise von Zuckeralkoholen, abgeleitet aus der Gruppe der reduzierenden Zucker oder reduzierenden Zuckerderivate. Bevorzugte reduzierende Zucker sind die Monosaccharide, vorzugsweise Pentosen und Hexosen, und die Oligosaccharide, vorzugsweise Disaccharide und gegebenenfalls auch Trisaccharide. Beispiele für Monosaccharide sind Glucose, Galaktose, Mannose und Talose als Hexosen und Arabinose, Ribose und Xylose als Pentosen. Von den Monosacchariden sind die Hexosen bevorzugt. Beispiele für Oligosaccharide (Polysaccharide) sind Lactose, Maltose, Maltotriose und dergleichen. Besonders bevorzugte Polyhydroxyalkylreste stammen von reduzierenden Hexosen, insbesondere von Glucose (Sorbitylrest).

Die erfindungsgemäßen Aminoxide der Formel (1) werden hergestellt durch Oxidation einer tertiären Aminverbindung der Formel (2) 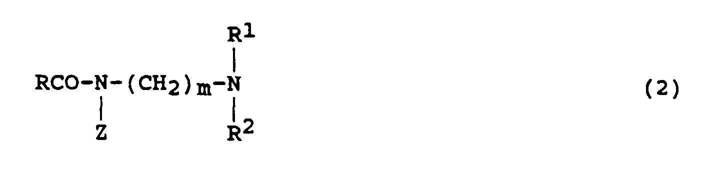 in der R, R¹, R², Z und m die angegebenen Bedeutungen haben,
mit Wasserstoffperoxid in Wasser, einem niedrigen Alkohol oder einer Mischung aus Wasser und einem niedrigen Alkohol als Lösungsmittel.

Die Umsetzung von tertiärer Aminverbindung, zum Beispiel N,N-Dimethylaminopropyl-Fettacyl-Glucamid, mit Wasserstoffperoxid wird im einzelnen in der Weise durchgeführt, daß man das tertiäre Amin und das Oxidationsmittel im Molverhältnis von 1 : 1 bis 1,2, vorzugsweise 1 : 1 bis 1,15, einsetzt; gegebenenfalls wird ein Sequestriermittel zugegeben. Das Lösungsmittel kann Wasser, ein niedriger Alkohol, vorzugsweise Methanol, Ethanol, Isopropanol, Ethylenglykol und/oder Propylenglykol oder eine Mischung aus Wasser und den genannten Alkoholen sein. Die Menge an Lösungsmittel (das als solches oder in Form von Lösungen der Ausgangsverbindungen in die Reaktionsmischung eingebracht wird) wählt man im allgemeinen so, daß die nach der Umsetzung erhaltene Aminoxidlösung einen Aminoxidgehalt (Wirkstoffgehalt) von 30 bis etwa 65 Gew.-% und vorzugsweise von 30 bis 60 Gew.-% hat, Gewichtsprozente bezogen auf die Lösung. Das Wasserstoffperoxid wird in Form von handelsüblichen wäßrigen Lösungen im Bereich von 20 bis 90 Gew.-% angewendet. Die Umsetzungstemperatur ist im allgemeinen 60 bis 110 °C, vorzugsweise 70 bis 100 °C. Die bei Atmosphärendruck ablaufende Oxidationsreaktion wird man so lange aufrechterhalten, bis der gewünschte Umsatz erreicht ist.

Die erhaltenen Aminoxidlösungen enthalten das erfindungsgemäße Aminoxid in hoher Konzentration. Es kann durch Abtrennen des Lösungsmittels in reiner Form gewonnen werden. Dies erübrigt sich im allgemeinen, weil die erfindungsgemäßen Aminoxide ohnehin vor allem in Lösungen zum Einsatz kommen.

Die zur Herstellung der erfindungsgemäßen Aminoxide und ihrer Lösungen erforderlichen Aminverbindungen der angegebenen Formel (2) erhält man vorteilhaft durch
a) Umsetzung von einer Polyhydroxykohlenwasserstoffverbindung, von der sich der Rest Z in Formel (1) oder Formel (2) ableitet, mit einem Amin der Formel (3) 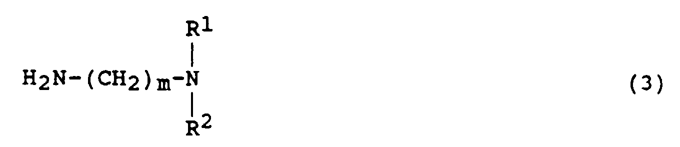 worin m, R¹ und R² die angegebenen Bedeutungen haben,
   in einem wäßrigen oder wäßrig-alkoholischen Medium und in Gegenwart von einem Hydrierkatalysator zum Polyhydroxyalkylamin der Formel (4) 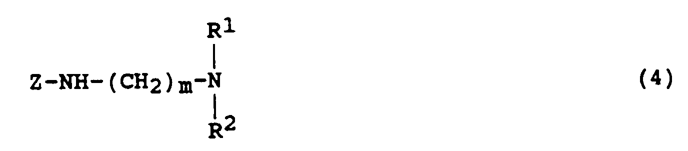 worin Z, m, R¹ und R² die genannten Bedeutungen haben,
   und
b) Umsetzung des im Schritt a) erhaltenen Produktes, bestehend im wesentlichen aus Polyhydroxyalkylamin der Formel (4), mit einem Fettsäurealkylester der Formel (5) 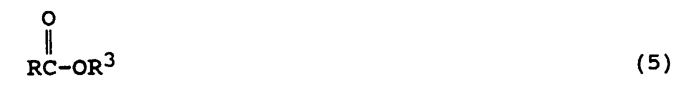 worin R die genannte Bedeutung hat und R³ eine C₁ bis C₃-Alkylgruppe ist,
   zum Polyhydroxyalkylamidamin der angegebenen Formel (2). Im folgenden werden die Schritte a) und b) noch näher beschrieben:

Der Schritt a) ist eine reduktive Aminierung von einer polyhydroxylierten Verbindung der oben angegebenen Art wie Mono- oder Disaccharidverbindungen, vorzugsweise Hexosen wie Glucose, mit einem Amin der Formel (3). Die Zuckerverbindung und die Aminverbindung werden im Molverhältnis von etwa 1 : 1 bis 1,2 eingesetzt. Das Lösungsmittel, das vorzugsweise Wasser oder eine Mischung aus Wasser und einem niedrigen Alkohol wie Methanol, Ethanol und/oder Isopropanol ist, wird in einer Menge von etwa 30 bis 50 Gew.-%, bezogen auf gebildetes Polyhydroxyalkylamin eingesetzt. Als Katalysatoren können die üblichen Hydrierungskatalysatoren wie Palladium auf Aktivkohle, Kupferchromit und insbesondere Raney-Nickel in einer Menge von im allgemeinen 0,01 bis 3 Gew.-%, vorzugsweise 0,1 bis 1 Gew.-%, bezogen auf die zu aminierende Zuckerverbindung, eingesetzt werden. Die reduktive Aminierungsreaktion wird bei einer Temperatur von 40 bis 150 °C, vorzugsweise 50 bis 120 °C, und bei einem Wasserstoffdruck von 10 bis 200 bar, vorzugsweise 20 bis 100 bar, durchgeführt. Die Aminozuckerverbindung gemäß Formel (4) fällt in praktisch quantitativen Ausbeuten an.

Im Schritt b) wird das im Schritt a) erhaltene Reaktionsprodukt (gegebenenfalls nach Abfiltrierung des Katalysators) in Gegenwart eines basischen Katalysators mit etwa 1 mol Fettsäureester der Formel (5) pro mol Aminozuckerverbindung acyliert. Dies wird vorzugsweise bei einer Temperatur von etwa 60 bis 130 °C durchgeführt, zum Beispiel durch Kochen der Reaktionsmischung am Rückfluß, und führt zum acylierten Aminozucker der Formel (2).

Die erfindungsgemäßen Aminoxide weisen unerwartet gute Eigenschaften auf. Sie sind in Wasser, niedrigen Alkoholen oder Mischungen davon bei Raumtemperatur (20 bis 25 °C) bis zu hohen Konzentrationen löslich. Die konzentrierten bis hochkonzentrierten Lösungen sind überraschenderweise bei Raumtemperatur niedrig viskos, das heißt gut fließbar, gießbar, pumpbar und dergleichen. Die erfindungsgemäßen wäßrigen, alkoholischen oder wäßrig-alkoholischen Aminoxidlösungen zeichnen sich ferner durch eine hohe Klarheit (sie erscheinen dem menschlichen Auge wasserklar) und Lagerbeständigkeit aus. Die erfindungsgemäßen Aminoxide beruhen auf nachwachsenden Rohstoffen und sind biologisch abbaubar, was einen weiteren Vorteil dieser Tensidverbindungen mit hervorragenden Tensideigenschaften darstellt. Aufgrund dieses Eigenschaftsbildes werden die erfindungsgemäßen Aminoxide und Aminoxidlösungen vorteilhaft zur Herstellung von oberflächenaktiven Mitteln zur Haar- und Körperpflege verwendet.

Die Erfindung wird nun an Beispielen noch näher erläutert, wobei die Abkürzungen "DMAP" Dimethylaminopropyl und "GA" Glucamid bedeuten.

### Beispiel 1 (DMAP-C₁₂-GA-Aminoxid)

In einem Vierhalskolben, ausgestattet mit Rückflußkühler, Rührer, Thermometer und Tropftrichter, werden 209,4 g (0,45 mol) eines 96gew.%igen alkali- und erdalkaliionenfreien beziehungsweise -armen DMAP-C₁₂-GA, 207,5 g destilliertes Wasser und 0,1 g Ethylendiamintetraessigsäure-dinatriumsalz (EDTA) vorgelegt und unter Rühren auf 70 °C erwärmt. Anschließend werden innerhalb 30 Minuten 45,9 g (0,473 mol) einer 35gew.%igen wäßrigen Wasserstoffperoxidlösung kontinuierlich zugetropft; durch die exotherme Reaktion steigt die Temperatur auf circa 80 °C. Das Reaktionsgemisch wird nun weitere 5 bis 8 Stunden bei 75 bis 80 °C gerührt und dabei eine dünnflüssige, 44 Gew.-% des entsprechenden Aminoxids der Formel (1) enthaltende Lösung erhalten (97 % Ausbeute); der Restgehalt an Wasserstoffperoxid liegt bei maximal 0,1 Gew.-%.

### Beispiel 2 (DMAP-C_{12/14}-GA-Aminoxid)

| Ansatzgröße: | |
|---|---|
| 263,0 g (0,55 mol) | eines 95gew.%igen alkali- und erdalkaliionenfreien beziehungsweise -armen DMAP-C_{12/14}-GA |
| 255,2 g | destilliertes Wasser |
| 0,1 g | EDTA |
| 56,1 g (0,578 mol) | einer 35gew.%igen wäßrigen Wasserstoffperoxidlösung |

Die Reaktion wird analog Beispiel 1 durchgeführt. Man erhält in 97%iger Ausbeute eine dünnflüssige, 44 Gew.-% des entsprechenden Aminoxids der Formel (1) enthaltende Lösung.

### Beispiel 3 (DMAP-C_{16/18}-GA-Aminoxid)

| Ansatzgröße: | |
|---|---|
| 138,0 g (0,25 mol) | eines 93gew.%igen alkali- und erdalkaliionenfreien beziehungsweise -armen DMAP-C_{16/18}-GA |
| 259,9 g | destilliertes Wasser |
| 0,1 g | EDTA |
| 25,5 g (0,263 mol) | einer 35gew.%igen wäßrigen Wasserstoffperoxidlösung |

Die Reaktion wird analog Beispiel 1 durchgeführt. Man erhält in 96%iger Ausbeute eine dünnflüssige, 30 Gew.-% des entsprechenden Aminoxids der Formel (1) enthaltende Lösung.

## Patentansprüche

1. Polyhydroxyalkyl-amidaminoxide der nachstehenden Formel (1) 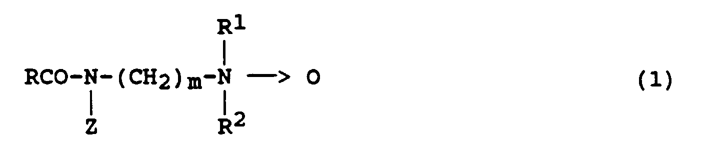 worin bedeuten
RCO einen aliphatischen Acylrest mit 6 bis 22 Kohlenstoffatomen,
Z einen linearen Polyhydroxykohlenwasserstoffrest mit mindestens 3 gegebenenfalls oxalkylierten Hydroxylgruppen,
m eine ganze Zahl von 1 bis 4,
R¹ C₁ bis C₄-Alkyl oder C₂ bis C₄-Hydroxyalkyl und
R² C₁ bis C₄-Alkyl oder C₂ bis C₄-Hydroxyalkyl.

2. Aminoxide nach Anspruch 1, wobei in Formel (1)
RCO ein Fettacylrest mit 8 bis 18 Kohlenstoffatomen ist,
Z ein Rest eines Zuckeralkohols ist, der sich von einem reduzierenden Mono- oder Disaccharid ableitet,
m für die Zahl 3 steht und
R¹ und R² Methyl, Ethyl, Propyl oder Hydroxyethyl sind.

3. Aminoxide nach Anspruch 1, wobei in Formel (1)
RCO ein Fettacylrest mit 8 bis 18 Kohlenstoffatomen ist,
Z ein Sorbitylrest ist,
m für die Zahl 3 steht und
R¹ und R² Methyl, Ethyl, Propyl oder Hydroxyethyl sind.

4. Verfahren zur Herstellung der Polyhydroxyalkyl-amidaminoxide nach Anspruch 1, gekennzeichnet durch Oxidation einer tertiären Aminverbindung der Formel (2) 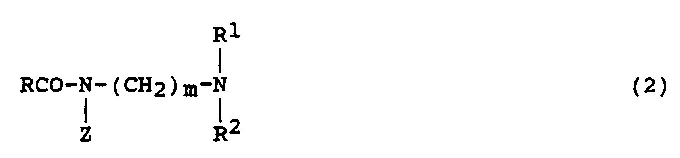 in der R, R¹, R², Z und m die angegebenen Bedeutungen haben,
mit Wasserstoffperoxid in Wasser, einem niedrigen Alkohol oder einer Mischung aus Wasser und einem niedrigen Alkohol als Lösungsmittel.

5. Verfahren nach Anspruch 4, dadurch gekennzeichnet, daß man 1 bis 1,2 mol Wasserstoffperoxid pro mol tertiäre Aminverbindung und das Lösungsmittel in einer solchen Menge einsetzt, daß die nach der Umsetzung erhaltene Aminoxidlösung einen Aminoxidgehalt von 30 bis 65 Gew.-% hat, bezogen auf das Gewicht der Lösung, und die Oxidationsreaktion bei einer Temperatur von 60 bis 110 °C durchgeführt wird.

6. Verfahren nach Anspruch 4, dadurch gekennzeichnet, daß man
a) eine Mischung aus dem Lösungsmittel und der tertiären Aminverbindung bereitet und diese Mischung auf 70 bis 100 °C erhitzt,
b) in die erhitzte Mischung 1 bis 1,2 mol Wasserstoffperoxid in Form einer 20 bis 90gew.%igen wäßrigen Lösung pro mol Amin unter Aufrechterhaltung der genannten Temperatur von 70 bis 100 °C einbringt und daß man
c) die im Schritt b) erhaltene Reaktionsmischung bei einer Temperatur von 70 bis 100 °C hält, bis der angestrebte Aminoxidgehalt erreicht ist.

7. Wäßrige, alkoholische oder wäßrig-alkoholische Lösungen von Polyhydroxyalkyl-amidaminoxiden, bestehend im wesentlichen aus
A) 30 bis 65 Gew.-% von mindestens einer Verbindung der Formel (1) in Anspruch 1 und
B) Wasser, einem niedrigen Alkohol oder einer Mischung aus Wasser und einem niedrigen Alkohol als Rest auf 100 Gew.-%.

8. Lösung nach Anspruch 7, dadurch gekennzeichnet, daß die Komponente A) in einer Menge von 30 bis 60 Gew.-% vorliegt.

9. Verwendung der Polyhydroxyalkyl-amidaminoxide nach Anspruch 1 zur Herstellung von oberflächenaktiven Mitteln zur Haar- und Körperpflege.

10. Verwendung der wäßrigen, alkoholischen oder wäßrigalkoholischen Lösungen nach Anspruch 7 zur Herstellung von oberflächenaktiven Mitteln zur Haar- und Körperpflege.

## Claims

1. A polyhydroxyalkyl-amidoamine oxide of the following formula (1) 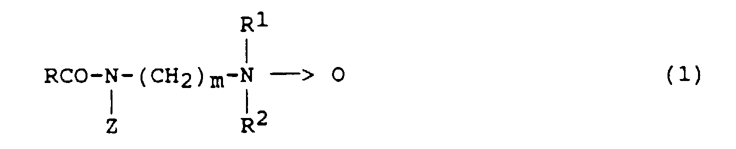 in which
RCO is an aliphatic acyl radical having 6 to 22 carbon atoms,
z is a linear polyhydroxyhydrocarbon radical having at least 3 optionally oxyalkylated hydroxyl groups,
m is an integer from 1 to 4,
R¹ is C₁ to C₄-alkyl or C₂ to C₄-hydroxyalkyl and
R² is C₁ to C₄-alkyl or C₂ to C₄-hydroxyalkyl.

2. An amine oxide as claimed in claim 1, in which, in formula (1),
RCO is a fatty acyl radical having 8 to 18 carbon atoms,
z is a radical of a sugar-alcohol which is derived from a reducing mono- or disaccharide,
m is the number 3 and
R¹ and R² are methyl, ethyl, propyl or hydroxyethyl.

3. An amine oxide as claimed in claim 1, in which, in formula (1),
RCO is a fatty acyl radical having 8 to 18 carbon atoms,
Z is a sorbityl radical,
m is the number 3 and
R¹ and R² are methyl, ethyl, propyl or hydroxyethyl.

4. A process for the preparation of a polyhydroxyalkyl-amidoamine oxide as claimed in claim 1, which comprises oxidation of a tertiary amine compound of the formula (2) 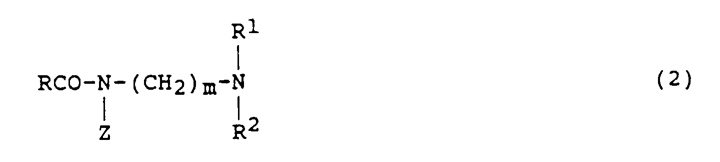 in which R, R¹, R², Z and m have the meanings given,
with hydrogen peroxide in water, a lower alcohol or a mixture of water and a lower alcohol as the solvent.

5. The process as claimed in claim 4, wherein 1 to 1.2 mol of hydrogen peroxide are employed per mole of tertiary amine compound and the solvent is employed in an amount such that the amine oxide solution obtained after the reaction has an amine oxide content of 30 to 65 % by weight, based on the weight of the solution, and the oxidation reaction is carried out at a temperature from 60 to 110°C.

6. The process as claimed in claim 4, wherein
a) a mixture of the solvent and the tertiary amine compound is prepared and this mixture is heated to 70 to 100°C,
b) 1 to 1.2 mol of hydrogen peroxide in the form of a 20 to 90 % strength by weight aqueous solution per mole of amine are introduced into the heated mixture, the said temperature of 70 to 100°C being maintained, and
c) the reaction mixture obtained in step b) is kept at a temperature of 70 to 100°C until the desired amine oxide content is reached.

7. An aqueous, alcoholic or aqueous-alcoholic solution of a polyhydroxyalkyl-amidoamine oxide, essentially comprising
A) 30 to 65 % by weight of at least one compound of the formula (1) in claim 1 and
B) water, a lower alcohol or a mixture of water and a lower alcohol as the remainder to make up to 100 % by weight.

8. A solution as claimed in claim 7, wherein component A) is present in an amount of 30 to 60 % by weight.

9. The use of a polyhydroxyalkyl-amidoamine oxide as claimed in claim 1 for the preparation of surface-active compositions for hair and body care.

10. The use of an aqueous, alcoholic or aqueous-alcoholic solution as claimed in claim 7 for the preparation of surface-active compositions for hair and body care.

## Revendications

1. Oxydes de polyhydroxyalkylamidamines de formule suivante (1) 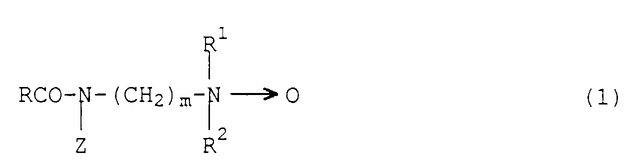 dans laquelle
RCO représente un groupe acyle aliphatique avec 6 à 22 atomes de carbone,
z représente un groupe linéaire polyhydroxyhydrocarboné avec au moins 3 groupes hydroxyle éventuellement alcoxylés,
m représente un nombre entier de 1 à 4,
R¹ un groupe alkyle en C₄ ou hydroxyalkyle en C₂ à C₄ et
R² un groupe alkyle en C₂ à C₄ ou hydroxyalkyle en C₂ à C₄.

2. Oxydes d'amines selon la revendication 1, dans lesquels dans la formule (1)
RCO est un groupe acyle gras avec 8 à 18 atomes de carbone,
z représente un résidu d'un alcool de sucre qui dérive d'un mono ou disaccharide réducteur,
m représente le nombre 3 et
R¹ et R² représentent un groupe méthyle, éthyle, propyle ou hydroxyéthyle.

3. Oxydes d'amines selon la revendication 1, dans lesquels dans la formule (1)
RCO est un groupe acyle gras avec 8 à 18 atomes de carbone,
z représente un groupe sorbityle,
m représente le nombre 3 et
R¹ et R² représentent un groupe méthyle, éthyle, propyle ou hydroxyéthyle.

4. Procédé de préparation d'oxydes de polyhydroxyalkylamidamines selon la revendication 1, caractérisé par l'oxydation d'un composé amine tertiaire de formule (2) 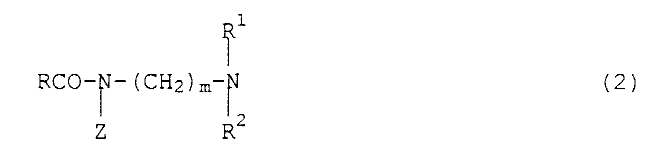 dans laquelle R, R¹, R², Z et m ont les significations mentionnées,
avec du peroxyde d'hydrogène dans l'eau, un alcool inférieur ou un mélange d'eau et d'un alcool inférieur comme solvant.

5. Procédé selon la revendication 4, caractérisé en ce qu'on utilise 1 à 1,2 mole de peroxyde d'hydrogène par mole de composé amine tertiaire et le solvant en une quantité telle que la solution d'oxyde d'amine contenue après la réaction a une teneur en oxyde d'amine de 30 à 65% en poids, par rapport au poids de la solution et en ce que la réaction d'oxydation est effectuée à une température de 60 à 110°C.

6. Procédé selon la revendication 4, caractérisé en ce qu'on réalise les étapes consistant
a) à préparer un mélange du solvant et du composé amine tertiaire et à chauffer ce mélange de 70 à 100°C,
b) à verser dans le mélange chaud 1 à 1,2 mole de peroxyde d'hydrogène, sous la forme d'une solution aqueuse de 20 à 90% en poids, par mole d'amine, en maintenant la température citée de 70 à 100°C et
c) à maintenir le mélange réactionnel obtenu à l'étape b) à une température de 70 à 100°C, jusqu'à l'obtention de la teneur en oxyde d'amine que l'on cherche à obtenir.

7. Solutions aqueuses, alcooliques ou aqueuses-alcooliques d'oxydes de polyhydroxyalkylamidamines, constituées essentiellement de
A) 30 à 65% en poids d'au moins un composé de formule (1) selon la revendication 1 et
B) d'eau, d'un alcool inférieur ou d'un mélange d'eau et d'un alcool inférieur comme complément à 100% en poids.

8. Solution selon la revendication 7, caractérisée en ce que le composant A) est présent en une quantité de 30 à 60% en poids.

9. Utilisation des oxydes de polyhydroxyalkylamidamines selon la revendication 1 pour la préparation d'agents tensioactifs pour les soins capillaires et corporels.

10. Utilisation des solutions aqueuses, alcooliques ou aqueuses-alcooliques selon la revendication 7 pour la préparation d'agents tensioactifs pour les soins capillaires et corporels.
